# EUROPÄISCHE PATENTSCHRIFT

(11) **EP 3 267 971 B1**
(45) Veröffentlichungstag und Bekanntmachung des Hinweises auf die Patenterteilung: **19.02.2020**
(21) Anmeldenummer: 15794543.7
(22) Anmeldetag: 11.11.2015
(51) Int. Cl.: A61K 8/81, A61Q 5/06

(54) **MITTEL UND VERFAHREN ZUR TEMPORÄREN VERFORMUNG KERATINHALTIGER FASERN**
PRODUCT AND METHOD FOR THE TEMPORARY SHAPING OF KERATIN-CONTAINING FIBERS
PRODUIT ET PROCÉDÉS DE MISE EN FORME TEMPORAIRE DE FIBRES KÉRATINIQUES

(30) Priorität: 09.03.2015 DE 102015204149
(43) Veröffentlichungstag der Anmeldung: 17.01.2018
(73) Patentinhaber: Henkel AG & Co. KGaA, 40589 Düsseldorf (DE)
(72) Erfinder: LANGE, Julia Bibiane, 24576 Bad Bramstedt (DE); PULS, Anna, 21423 Winsen (Luhe) (DE); MARTINEZ, Cyrielle, 22765 Hamburg (DE); RICHTERS, Bernd, 21129 Hamburg (DE)
(86) Internationale Anmeldenummer: PCT/EP2015/076326
(87) Internationale Veröffentlichungsnummer: WO 2016/142010

(56) Entgegenhaltungen:
- EP-A1- 1 238 646
- EP-A2- 1 878 423
- DE-A1-102007 008 089
- "Acrylates copolymers-Why do we need to neutralize them", Cosmetic and science Technology, 1. Januar 2006 (2006-01-01), Seite 219, XP055236812, Gefunden im Internet: URL:http://cosmeticsciencetechnology.com/a rticles/samples/891.pdf [gefunden am 2015-12-16]
- JONES C: "Multifunctional Synthetic Rheology Modifiers for Personal Care Formulations: More Than Just Thickeners", ROHM AND HAAS PERSONAL CARE,, 1. Mai 2005 (2005-05-01), Seiten 1-23, XP007909943,
- BASF: "Acrylic terpolymer products for hair-setting preparations with a strong, long-lasting effect (Luvimer)", TECHNICAL INFORMATION BASF,, 1. September 2000 (2000-09-01), Seiten 1-21, XP007921915,

## Beschreibung

Die vorliegende Erfindung betrifft eine kosmetische Zusammensetzung zur Haarfestigung bzw. zur temporären Umformung von keratinischen Fasern, insbesondere menschlichen Haaren, wobei die Zusammensetzung eine Kombination von zwei anionischen Acrylatpolymeren enthält.

Die temporäre Gestaltung von Frisuren für einen längeren Zeitraum bis hin zu mehreren Tagen erfordert in der Regel die Anwendung festigender Wirkstoffe. Daher spielen Haarbehandlungsmittel, die einer temporären Formgebung der Haare dienen, eine wichtige Rolle. Entsprechende Mittel zur temporären Verformung enthalten als festigenden Wirkstoff üblicherweise synthetische Polymere und/oder Wachse. Mittel zur Unterstützung der temporären Umformung keratinhaltiger Fasern können beispielsweise als Haarspray, Haarwachs, Haargel, Haarschaum konfektioniert werden.

Die wichtigste Eigenschaft eines Mittels zur temporären Verformung von Haaren, im Folgenden auch Stylingmittel genannt, besteht darin, den behandelten Fasern in der neu modellierten Form - d.h. einer den Haaren aufgeprägten Form - einen möglichst starken Halt zu geben. Man spricht auch von starkem Frisurenhalt oder vom hohen Haltegrad des Stylingmittels. Der Frisurenhalt wird im Wesentlichen durch die Art und Menge des eingesetzten festigenden Wirkstoffe bestimmt, wobei jedoch auch ein Einfluss der weiteren Bestandteile des Stylingmittels gegeben sein kann.

Neben einem hohen Haltegrad müssen Stylingmittel eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit (tack) und ein ausgewogener Konditioniereffekt. Weiterhin soll ein Stylingmittel möglichst für alle Haartypen universell einsetzbar und mild zu Haar und Haut sein.

Um den unterschiedlichen Anforderungen gerecht zu werden, wurde als festigende Wirkstoffe bereits eine Vielzahl von synthetischen Polymeren entwickelt, die in Stylingmitteln zur Anwendung kommen. Die Polymere lassen sich in kationische, anionische, nichtionische und amphotere festigende Polymere unterteilen. Idealerweise ergeben die Polymere bei der Anwendung auf dem Haar einen Polymerfilm, der einerseits der Frisur einen starken Halt verleiht, andererseits aber hinreichend flexibel ist, um bei Beanspruchung nicht zu brechen. Ist der Polymerfilm zu brüchig, kommt es zur Bildung so genannter Filmplaken, das heißt Rückständen, die sich bei der Bewegung des Haares ablösen und den Eindruck vermitteln, der Anwender des entsprechenden Stylingmittels hätte Schuppen. Ähnliche Probleme ergeben sich, wenn Wachse als festigender Wirkstoff im Stylingmittel Einsatz finden. Handelt es sich bei dem Stylingmittel um ein Gel oder eine Paste, sollen die Polymere zudem verdickende Eigenschaften besitzen.

Bekannte anionische Polymere, die in Haarfestigungsprodukten Anwendung finden, sind Acrylatcopolymere mit zwei oder mehr Struktureinheiten. Bestimmte derartige Copolymere mit der Handelsbezeichnung Aculyn® 33A (INCI: Acrylates Copolymer) und deren Einsatz in kosmetischen Zusammensetzungen zur temporären Verformung keratinischer Fasern, werden in der deutschen Anmeldung DE 10 2007 053 954 A1 beschrieben.

Die DE102007008089 A1 offenbart eine kosmetische Zubereitung, insbesondere ein Stylinggel, enthaltend a) mindestens ein anionisches filmbildendes Polymer, b) mindestens ein hydrophob modifiziertes Acrylatverdicker und c) mindestens ein Block-Polymer.

EP 1 238 646 A1 befasst sich mit Haarbehandlungsmittel in Form eines wässrigen Gels mit verbesserten rheologischen Eigenschaften. Das Mittel enthält eine Kombination aus einem filmbildenden Copolymers, umfassend mindestens ein C₁-C₆-Alkyl(meth)acrylat und mindestens eine α,β-ungesättigten Carbonsäure.

Die EP 1 878 423 A2 offenbart kosmetische Zubereitungen, insbesondere Haarstylingzubereitungen, enthaltend Proteinhydrolysate aus Kaschmir-Wolle und fixierende Polymere.

Weiterhin sind hydrophob modifizierte Acrylatcopolymere (INCI: Acrylates Copolymer (and) Water) im Handel erhältlich, die im Wesentlichen als Verdickungsmittel wirken. Das Datenblatt AquaStyle® SH-100 Polymer (Ashland Inc.) beschreibt ein solches Acrylatcopolymer und dessen Verwendung in Kombination mit Carbomeren. Es wird eine Eignung für kristallklare Haargele, eine gute Anfangssteifheit, Feuchtigkeitsbeständigkeit und eine Langzeitwirkung beschrieben.

Eine Aufgabe der vorliegenden Erfindung war es, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen. Insbesondere sind derzeit erhältliche Stylingmittel noch dahingehend verbesserbar, dass eine gute Kombination aus Steifheit (Stiffness) und Langzeithalt (High Humidity Curl Retention) nicht immer ausreichend gewährleistet wird. Es ist daher eine Aufgabe der vorliegenden Erfindung, derartige Stylingmittel bereitzustellen, die neben den oben genannten Eigenschaften insbesondere sowohl eine gute Steifheit als auch einen guten Langzeithalt ergeben.

Dies wurde erfindungsgemäß durch eine Kombination zweier bestimmter, voneinander verschiedener anionischer Acrylatpolymere erreicht.

Durch die vorliegende Erfindung wird bereitgestellt:
1. Eine kosmetische Zusammensetzung zur temporären Umformung keratinischer Fasern, welche enthält:
   (a) mindestens ein Acrylatcopolymer (a), welches zumindest aus folgenden Monomereinheiten aufgebaut ist:
      (a1) mindestens eine (Meth)Acrylsäureeinheit
      (a2) mindestens eine (Meth)Acrylsäureestereinheit, welche einen Acrylsäure-(C1-4)-alkylester aufweist,
      und
   (b) mindestens ein von dem Acrylatcopolymer (a) verschiedenes anionisches Acrylatcopolymer (b), welches nur aus den Einheiten (b1), (b2) und (b3) aufgebaut ist, wobei
      (b1) mindestens eine (Meth)Acrylsäureeinheit, welche Methacrylsäure aufweist,
      (b2) mindestens eine (Meth)Acrylsäureethylestereinheit, welche Ethylacrylat aufweist, und
      (b3) mindestens eine (Meth)Acrylsäureestereinheit, die von der (Meth)Acrylsäureethylestereinheit (b2) verschieden und ein (Meth)Acrylsäurealkylester mit einer linearen oder verzweigten Alkylgruppe mit 2 bis 12 Kohlenstoffatomen ist,
      aufweist.
2. Kosmetische Zusammensetzung nach Punkt 1, wobei das mindestens eine Acrylatcopolymer (a) bezogen auf sein Gesamtgewicht zu mindestens 90 Gew.-%, vorzugsweise zu mindestens 95 Gew.-% und insbesondere zu mindestens 97 Gew.-% aus den Monomeren (a1) (Meth)Acrylsäureinheit und (a2) (Meth)Acrylsäureestereinheit besteht.
3. Kosmetische Zusammensetzung nach einem der vorhergehenden Punkte, wobei die Zusammensetzung das Copolymer (a) in einem Anteil von 0,05 bis 5,0 Gew.-%, bevorzugt 0,5 bis 4,0 Gew.-%, weiterhin bevorzugt 1,0 bis 3,0 Gew.-% enthält, bezogen auf das Gesamtgewicht der kosmetischen Zusammensetzung.
4. Kosmetische Zusammensetzung nach einem der vorhergehenden Punkte, wobei die Zusammensetzung das anionische Acrylatcopolymer (b) in einem Anteil von 0,05 bis 5,0 Gew.-%, bevorzugt 0,5 bis 4,0 Gew.-%, weiterhin bevorzugt 1,0 bis 3,0 Gew.-% enthält, bezogen auf das Gesamtgewicht der kosmetischen Zusammensetzung.
5. Kosmetische Zusammensetzung nach einem der vorhergehenden Punkte, wobei das anionische Acrylatcopolymer (b) bei einem Feststoffgehalt von 2 Gew.-% in einer wässerigen neutralisierten Lösung bei 25°C eine Viskosität von 60000 bis 120000 cPs aufweist.
6. Kosmetische Zusammensetzung einem der vorhergehenden Punkte, wobei das anionische Acrylatcopolymer (a) eines mit der INCI-Bezeichnung Acrylates Copolymer, insbesondere Aculyn® 33A (Rohm&Haas), ist.
7. Kosmetische Zusammensetzung einem der vorhergehenden Punkte, wobei das anionische Acrylatcopolymer (b) eines mit der INCI-Bezeichnung Acrylates Copolyme (and) Water, insbesondere AquaStyle SH-100 (Ashland Inc.), ist.
8. Kosmetische Zusammensetzung einem der vorhergehenden Punkte, wobei das anionische Acrylatcopolymer (a) eines mit der INCI-Bezeichnung Acrylates Copolymer und das anionische Acrylatcopolymer (b) eines mit der INCI-Bezeichnung Acrylates Copolymer (and) Water ist.
9. Kosmetische Zusammensetzung einem der vorhergehenden Punkte, wobei das anionische Acrylatcopolymer (a) Aculyn® 33A (Rohm&Haas) ist und das anionische Acrylatcopolymer (b) AquaStyle® SH-100 (Ashland Inc.) ist.
10. Kosmetische Zusammensetzung nach einem der vorhergehenden Punkte, welche, bezogen auf das Gesamtgewicht der kosmetischen Zusammensetzung, enthält:
   0,05 bis 5,0 Gew.-% des anionischen Acrylatcopolymers (a), und
   0,05 bis 5,0 Gew.-% des anionischen Acrylatcopolymers (b).
11. Kosmetische Zusammensetzung nach einem der vorhergehenden Punkte, enthaltend, bezogen auf das Gesamtgewicht der kosmetischen Zusammensetzung:
   1,0 bis 3,0 Gew.-% des anionischen Acrylatcopolymers (a), und
   1,0 bis 3,0 Gew.-% des anionischen Acrylatcopolymers (b).
12. Kosmetische Zusammensetzung einem der vorhergehenden Punkte, wobei die Zusammensetzung weiterhin mindestens ein von den Acrylatcopolymeren (a) und (b) verschiedenes Polymer (c), insbesondere ein anionisches oder nichtionisches Polymer (c), enthält.
13. Kosmetische Zusammensetzung nach einem der vorherigen Punkte, dadurch gekennzeichnet, dass es bezogen auf sein Gesamtgewicht weiterhin
   c) 1,0 bis 10 Gew.-% Polyvinylpyrrolidon und/oder Vinylpyrrolidon/Vinylacetat-Copolymer, vorzugsweise Polyvinylpyrrolidon enthält.
14. Kosmetische Zusammensetzung nach Punkt 13, dadurch gekennzeichnet, dass der Gewichtsanteil des Polyvinylpyrrolidons und/oder Vinylpyrrolidon/Vinylacetat-Copolymers c) am Gesamtgewicht der kosmetische Zusammensetzung 2,0 bis 8,5 Gew.-%, vorzugsweise 3,0 bis 7,0 Gew.-% beträgt.
15. Kosmetische Zusammensetzung nach einem der vorhergehenden Punkte, wobei die Zusammensetzung Wasser in einem Anteil von 50 und 95 Gew.-%, bevorzugt zwischen 60 und 90 Gew.-% und insbesondere zwischen 65 und 85 Gew.-%enthält, bezogen auf das Gesamtgewicht der kosmetischen Zusammensetzung.
16. Kosmetische Zusammensetzung nach einem der vorhergehenden Punkte, wobei die Zusammensetzung als Haargel, Haarspray, Haarschaum oder Haarwachs, insbesondere als Haargel, vorliegt.
17. Verwendung einer kosmetischen Zusammensetzung nach einem der Punkte 1 bis 16 zur temporären Umformung keratinischer Fasern.
18. Verwendung einer kosmetischen Zusammensetzung nach einem der Punkte 1 bis 16 zur Verbesserung der Feuchtebeständigkeit temporär verformter keratinischer Fasern.
19. Verfahren zur temporären Verformung von keratinischen Fasern, insbesondere menschlichen Haaren, bei dem die kosmetische Zusammensetzung nach einem der Punkte 1 bis 16 auf keratinische Fasern appliziert wird.

Es wurde im Rahmen der vorliegenden Erfindung überraschend festgestellt, dass durch Kombination zweier an sich bekannter Bestandteile, die in Stylingprodukten bereits Anwendung finden, eine verbesserte Feuchtebeständigkeit von Stylingprodukten erhalten werden kann. Andere üblicherweise geforderte Eigenschaften von Stylingprodukten wie Langzeithalt, Steifheit und geringe Klebrigkeit blieben dabei erhalten. Eine derartige gute Kombination von Eigenschaften war selbst bei Kenntnis der Einzelkomponenten nicht zu erwarten und war überraschend. Es zeigte sich experimentell, dass durch die Kombination der beiden Komponenten ein stark überadditiver, also synergistischer Effekt hinsichtlich der Feuchtebeständigkeit erhalten wurde, was sich im HHRC-Test (High Humidity Curl Retention-Test) manifestierte.

Der Begriff keratinische Fasern umfasst erfindungsgemäß Pelze, Wolle und Federn, insbesondere aber menschliche Haare.

Die wesentlichen Bestandteile der erfindungsgemäßen kosmetischen Zusammensetzung sind das anionisches Acrylatcopolymer (a) und das von dem Acrylatcopolymer (a) verschiedene anionische Acrylatcopolymer (b).

Das anionische Acrylatcopolymer (a) ist zumindest aus mindestens einer (Meth)Acrylsäureeinheit (a1) und mindestens einer (Meth)Acrylsäureestereinheit (a2) aufgebaut.
Das Copolymer (a) kann erfindungsgemäß aus weiteren Monomereinheiten aufgebaut sein. In einer bevorzugten Ausführungsform der Erfindung ist das Copolymer (a) aber nur aus den Einheiten (a1) und (a2) aufgebaut, d. h. es besteht aus von diesen Monomereinheiten abgeleiteten Einheiten.

Die mindestens eine (Meth)Acrylsäureeinheit (a1) kann eine Methacrylsäure- oder Acrylsäureeinheit sein, wobei eine Acrylsäureeinheit bevorzugt ist.

Die mindestens eine (Meth)Acrylsäureestereinheit (a2) weist eine Acrylsäure-(C1-4)-alkylestereinheit auf.

Ganz besonders bevorzugte Struktureinheiten (a2) sind Acrylsäuremethylester, Acrylsäureethylester, Acrylsäurepropylester, Acrylsäureisopropylester, und Acrylsäurebutylester.

Geeignete anionische Acrylatcopolymere (a) sind im Handel unter der INCI-Bezeichnung Acrylates Copolymer erhältlich. Am bevorzugtesten ist das anionische Acrylatcopolymer (a) Aculyn® 33A von Rohm&Haas. Dieses hat in der im Handel erhältlichen Form einen Feststoffgehalt von etwa 27,5 bis 28,5 Gew.-% und einen pH-Wert von 2,1 bis 3,5.

Die erfindungsgemäßen kosmetischen Zusammensetzungen enthalten als zweiten wesentlichen Bestandteil ein anionisches Acrylatcopolymer (b).

Das anionische Acrylatcopolymer (b) besteht aus den folgenden Monomereinheiten: mindestens einer (Meth)Acrylsäureeinheit (b1), welche Methacrylsäure aufweist, mindestens einer (Meth)Acrylsäureethylestereinheit (b2), welche Ethylacrylat aufweist, und mindestens einer (Meth)Acrylsäureestereinheit (b3), die von der (Meth)Acrylsäureethylestereinheit (b2) verschieden und ein (Meth)Acrylsäurealkylester mit einer linearen oder verzweigten Alkylgruppe mit 2 bis 12 Kohlenstoffatomen ist..

Die mindestens eine (Meth)Acrylsäureeinheit (b1) kann bevorzugt eine Methacrylsäureeinheit sein.

Die mindestens eine (Meth)Acrylsäureethylestereinheit (b2) kann eine Methacrylsäureethylestereinheit oder eine Acrylsäureethylestereinheit, wobei eine Acrylsäureethylestereinheit bevorzugt ist.

Die mindestens eine (Meth)Acrylsäureestereinheit (b3ist eine (Meth)Acrylsäurealkylestereinheit sein. Die Alkylgruppe der (Meth)Acrylsäurealkylestereinheit dient zur Steuerung der Hydrophobizität des Copolymers. Die Alkylgruppe ist eine lineare oder verzweigte Alkylgruppe mit 2 bis 12 Kohlenstoffatomen.

Die Viskosität des in der kosmetischen Zusammensetzung verwendeten anionischen Acrylatcopolymers (b) bei einem Feststoffgehalt von 2 Gew.-% und neutralisierter Lösung bei 25°C ist bevorzugt höchstens 60000 bis 120000 cPS.

Geeignete anionische Acrylatcopolymere (b) sind im Handel unter der INCI-Bezeichnung Acrylates Copolymer (and) Water erhältlich. Am bevorzugtesten ist das anionische Acrylatcopolymer (b) AquaStyle® SH-100 Polymer von Ashland, Inc. Dieses hat in der im Handel erhältlichen Form einen Feststoffgehalt von etwa 28 bis 32 Gew.-% und einen pH-Wert von 2,1 bis 4,0.

Die kosmetische Zusammensetzung der vorliegenden Erfindung enthält das Acrylatcopolymer (a) und Acrylatcopolymer (b) in für Stylingmittel üblichen und geeigneten Mengen, die für die spezielle Anwendung und Konfektionierung angepasst werden können.

Die erfindungsgemäße Zusammensetzung kann das Copolymer (a) beispielsweise in einer Menge von 0,05 bis 5,0 Gew.-%, bezogen auf das Gesamtgewicht der erfindungsgemäßen Zusammensetzung, enthalten. Bevorzugter sind Anteile des Copolymer (a) von 0,5 bis 4,0 Gew.-% und insbesondere von 1,0 bis 3,0 Gew.-%, jeweils angegeben als Feststoffgehalt aktiver Substanz in der kosmetischen Zusammensetzung.

Die erfindungsgemäße kosmetische Zusammensetzung enthält das Copolymer (b), bezogen auf das Gesamtgewicht der kosmetischen Zusammensetzung, z. B. in einer Menge von 0,05 bis 5,0 Gew.-%, bevorzugt 0,5 bis 4,0 Gew.-%, weiterhin bevorzugt 1,0 bis 3,0 Gew.-%, jeweils angegeben als Feststoffgehalt aktiver Substanz in der kosmetischen Zusammensetzung.

Die erfindungsgemäßen kosmetischen Zusammensetzungen zeichnen sich gegenüber alternativen kosmetischen Mitteln neben den oben genannten Vorteilen insbesondere auch durch einen verbesserten Langzeithalt aus. Als für die kosmetischen Eigenschaften der erfindungsgemäßen Mittel besonders vorteilhaft hat sich ein Gewichtsverhältnis der Polymere a) und b) in dem kosmetischen 5:1 bis 1:5, vorzugsweise 3:1 bis 1:3 und insbesondere 2:1 bis 1:2 erwiesen.

In einer besonders bevorzugten Ausführungsform der vorliegenden Erfindung enthält die kosmetische Zusammensetzung als das anionische Acrylatcopolymer (a) das im Handel unter der Bezeichnung Aculyn® 33A erhältliche Copolymer und als das anionische Acrylatcopolymer (b) das im Handel unter der Bezeichnung AquaStyle® SH-100 erhältliche Copolymer. Bei dieser Kombination wurden besonders gute Ergebnisse hinsichtlich einer Kombination von Steifheit und Langzeithalt erzielt. Besonders vorteilhaft ist diese Polymerkombination bei Stylingprodukten in Gelform.
Weitere allgemein geforderte Eigenschaften von Stylingprodukten, wie z. B. Feuchtebeständigkeit und niedrige Klebrigkeit, werden insbesondere mit dieser Kombination gleichfalls erzielt, besonders bei Konfektionierung als Haargel.

Die Acrylatcopolymere (a) und (b) werden in der kosmetischen Zusammensetzung vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem C₂-C₆-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten daher 2-Amino-2-methylpropanol. Das 2-Amino-2-methylpropanol wird in den erfindungsgemäßen Mitteln vorzugsweise in einer Menge eingesetzt, welche die zur Neutralisation der Acrylatcopolymere (a) und (b) benötigte Menge nicht überschreitet. Vorzugsweise beträgt die in den erfindungsgemäßen Zusammensetzungen eingesetzte Mengen an 2-Amino-2-methylpropanol 80 bis 100%, besonders bevorzugt 90 bis 100% und insbesondere 95 bis 100% der zur vollständigen Neutralisation der Acrylatcopolymere (a) und (b) benötigten Menge. In einer bevorzugten Ausführungsform beträgt der Gewichtsanteil des 2-Amino-2-methylpropanols am Gesamtgewicht des kosmetischen Mittels 0,05 bis 7,0 Gew.-%, bevorzugt 0,1 bis 5,0 Gew.% und insbesondere 0,1 bis 3,0 Gew.-%.

Zusammenfassend enthält eine bevorzugte kosmetische Zusammensetzung zur temporären Umformung keratinischer Fasern bezogen auf ihr Gesamtgewicht:
(a) 0,5 bis 4,0 Gew.-% mindestens eines quervernetzten Copolymers (a), welches zumindest aus folgenden Monomereinheiten aufgebaut ist:
   (a1) mindestens eine (Meth)Acrylsäureeinheit
   (a2) mindestens eine (Meth)Acrylsäureestereinheit, welche einen Acrylsäure-(C1-4)-alkylester aufweist,
   und
(b) 0,5 bis 4,0 Gew.-% mindestens eines anionischen Copolymers (b), welches nur aus den Einheiten (b1), (b2) und (b3) aufgebaut ist, wobei
   (b1) mindestens eine (Meth)Acrylsäureeinheit, welche Methacrylsäure aufweist,
   (b2) mindestens eine (Meth)Acrylsäureethylestereinheit, welche Ethylacrylat aufweist, und
   (b3) mindestens eine (Meth)Acrylsäureestereinheit, die von der (Meth)Acrylsäureethylestereinheit (b2) verschieden und ein (Meth)Acrylsäurealkylester mit einer linearen oder verzweigten Alkylgruppe mit 2 bis 12 Kohlenstoffatomen ist, aufweist.

Bevorzugt enthält die kosmetische Zusammensetzung der vorliegenden Erfindung eine oder mehrere weitere, als Verdickungsmittel oder Gelbildner wirkende Komponente(n), die von dem Acrylatcopolymer (a) und (b) verschieden ist/sind und ebenfalls die Filmbildung unterstützen. Beispiele sind kationische, anionische, nichtionische oder amphotere Polymere. Der Gewichtsanteil dieser weiteren Komponente am Gesamtgewicht der kosmetischen Zusammensetzung kann aufgrund der Anwesenheit der Komponenten (a) und (b) vergleichsweise niedrig sein und beträgt beispielsweise 0,02 bis 3 Gew.-%, bevorzugt 0,05 bis 1,5 Gew.-% und noch bevorzugter 0,2 bis 0,8 Gew.-%.

Beispiele sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Beispiele für nichtionische Polymere sind:
- Vinylpyrrolidon/Vinylester-Copolymere, wie sie beispielsweise unter dem Warenzeichen Luviskol (BASF) vertrieben werden. Luviskol VA 64 und Luviskol VA 73, jeweils Vinylpyrrolidon/VinylacetatCopolymere, sind bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminalund Benecel (AQUALON) vertrieben werden.
- Schellack.
- Polyvinylpyrrolidone, wie sie beispielsweise unter der Bezeichnung Luviskol (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder Hydroxy-Gruppen enthalten.
- Glycosidisch substituierte Silicone.

Bevorzugt ist die weitere als Gelbildner wirkende Komponente eine Homopolyacrylsäure (INCI: Carbomer), die im Handel unter dem Namen Carbopol® in unterschiedlichen Ausführungen erhältlich ist. Das Carbomer ist bevorzugt in einem Anteil von 0,02 bis 3 Gew.-%, bevorzugt 0,05 bis 1,5 Gew.-% und noch bevorzugter 0,2 bis 0,8 Gew.-%, in Bezug auf das Gesamtgewicht der kosmetischen Zusammensetzung, enthalten.

Aufgrund ihrer kosmetischen Wirkung in Kombination mit den Copolymeren a) und b) erfindungsgemäß bevorzugt eingesetzte filmbildenden Polymere sind insbesondere die Polyvinylpyrrolidone (INCI-Bezeichnung: PVP) sowie die Vinylpyrrolidon/Vinylacetat-Copolymere (INCI-Bezeichnung VP/VA Copolymer), wobei der Gewichtsanteil dieser Polymere vorzugsweise auf Mengen zwischen 1,0 und 10 Gew.-% beschränkt wird. Besonders bevorzugte erfindungsgemäße kosmetische Zusammensetzungen sind daher dadurch gekennzeichnet, dass sie bezogen auf ihr Gesamtgewicht weiterhin 1,0 bis 10 Gew.-% Polyvinylpyrrolidon und/oder Vinylpyrrolidon/Vinylacetat-Copolymer, vorzugsweise Polyvinylpyrrolidon enthalten. Besonders bevorzugte kosmetische Mittel weisen einen Gewichtsanteil des Polyvinylpyrrolidons und/oder Vinylpyrrolidon/Vinylacetat-Copolymers c) am Gesamtgewicht des kosmetischen Mittels von 2,0 bis 8,5 Gew.-%, vorzugsweise von 3,0 bis 7,0 Gew.-% auf.

Die erfindungsgemäße kosmetische Zusammensetzung kann weitere übliche Stoffe von Stylingprodukten enthalten. Als weitere geeignete Hilfs- und Zusatzstoffe sind insbesondere zusätzliche Pflegestoffe zu nennen.

Als Pflegestoff kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von 6-C30-Fettsäuren mit C2-C30-Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

Weiterhin sind in der erfindungsgemäßen Zusammensetzung bevorzugt Emulgatoren bzw. oberflächenaktive Mittel enthalten. Bevorzugt sind PEG-Derivate von hydriertem Ricinusöl, die z. B. unter der Bezeichnung PEG Hydrogenated Castor Oil erhältlich sind, z.B. PEG-30 Hydrogenated Castor Oil, PEG-33 Hydrogenated Castor Oil, PEG-35 Hydrogenated Castor Oil, PEG-36 Hydrogenated Castor Oil oder PEG-40 Hydrogenated Castor Oil. Erfindungsgemäß bevorzugt ist die Verwendung von PEG-40 Hydrogenated Castor Oil. Diese sind bevorzugt in einer Menge von 0,05 bis 1,5 Gew.-% enthalten, bevorzugter 0,1 bis 1,0 Gew.-%, ebenfalls bevorzugt 0,2 bis 0, 8 Gew.-% oder 0,3 bis 0,6 Gew.-%.

Die erfindungsgemäßen kosmetischen Mittel enthalten die Inhalts- bzw. Wirkstoffe in einem kosmetisch akzeptablen Träger.

Bevorzugte kosmetisch akzeptable Träger sind wässrige, alkoholische oder wässrig-alkoholische Medien mit vorzugsweise mindestens 10 Gew.-% Wasser, berechnet auf das Gesamtgewicht des Mittels.

Besonders bevorzugt enthält der erfindungsgemäße kosmetische Träger Wasser, insbesondere in der Menge, dass das kosmetische Mittel, berechnet auf das Gesamtgewicht des Mittels, mindestens 10 Gew.-%, insbesondere mindestens 20,0 Gew.-%, am bevorzugtesten mindestens 40 Gew.-% Wasser enthält. Ganz besonders bevorzugte kosmetische Mittel weisen bezogen auf ihre Gesamtgewicht einen Wasseranteil zwischen 50 und 95 Gew.-%, bevorzugt zwischen 60 und 90 Gew.-% und insbesondere zwischen 65 und 85 Gew.-% auf.

Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein.

Beispiele für wasserlösliche Lösungsmittel als Cosolvens sind Glycerin und/oder Ethylenglykol und/oder 1,2-Propylenglykol in einer Menge von 0 bis 30 Gew.-% bezogen auf das gesamte Mittel.

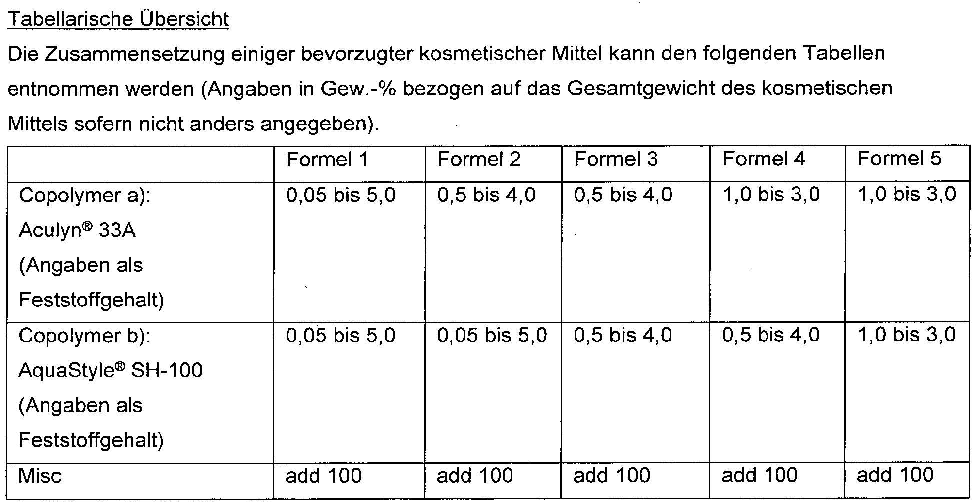

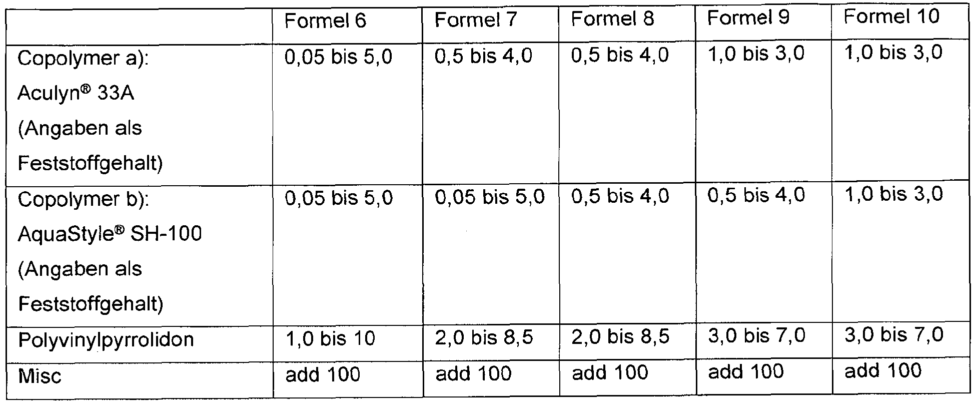

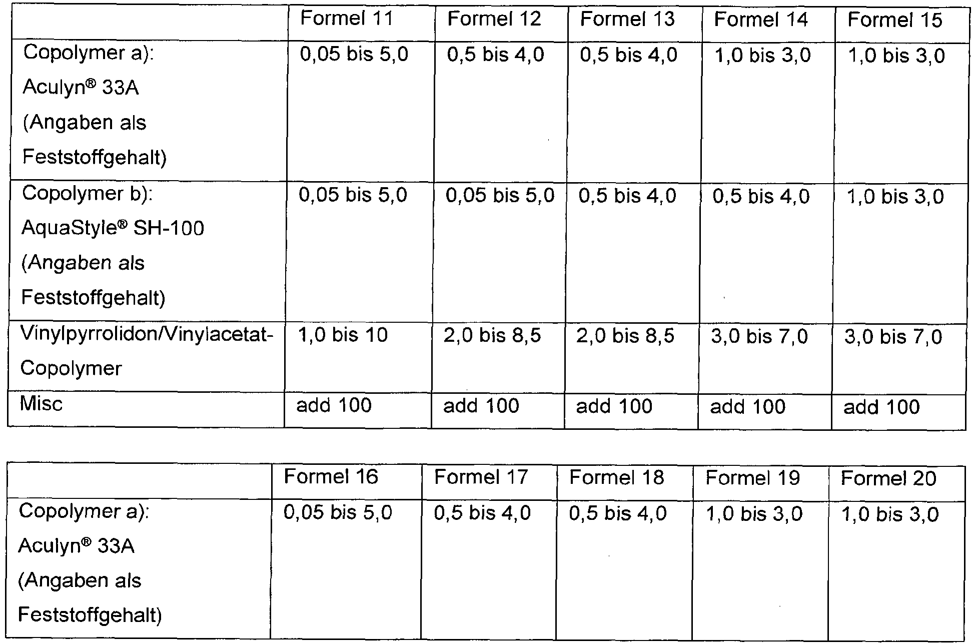

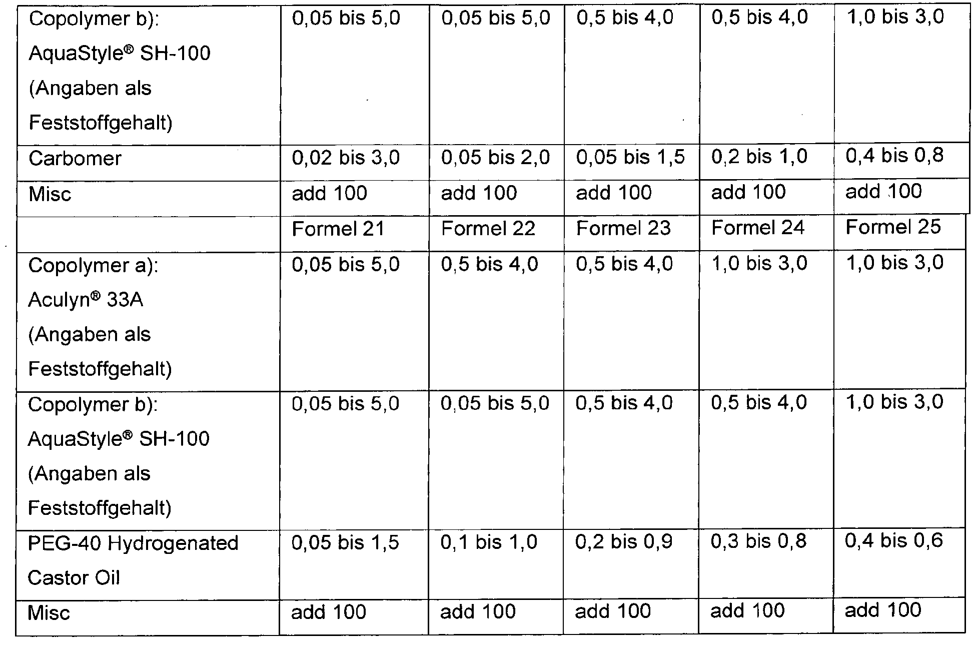

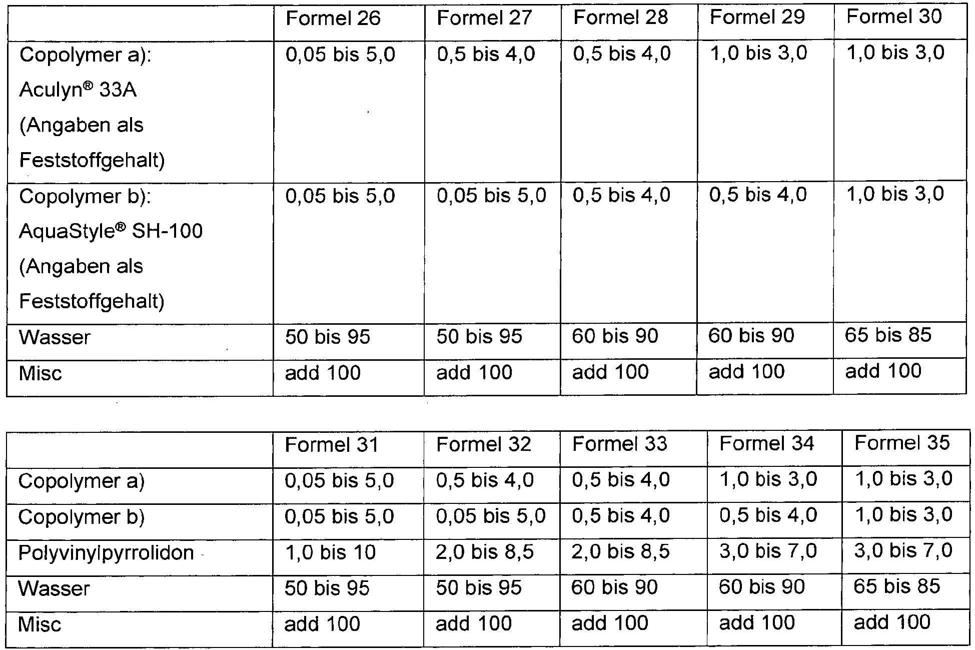

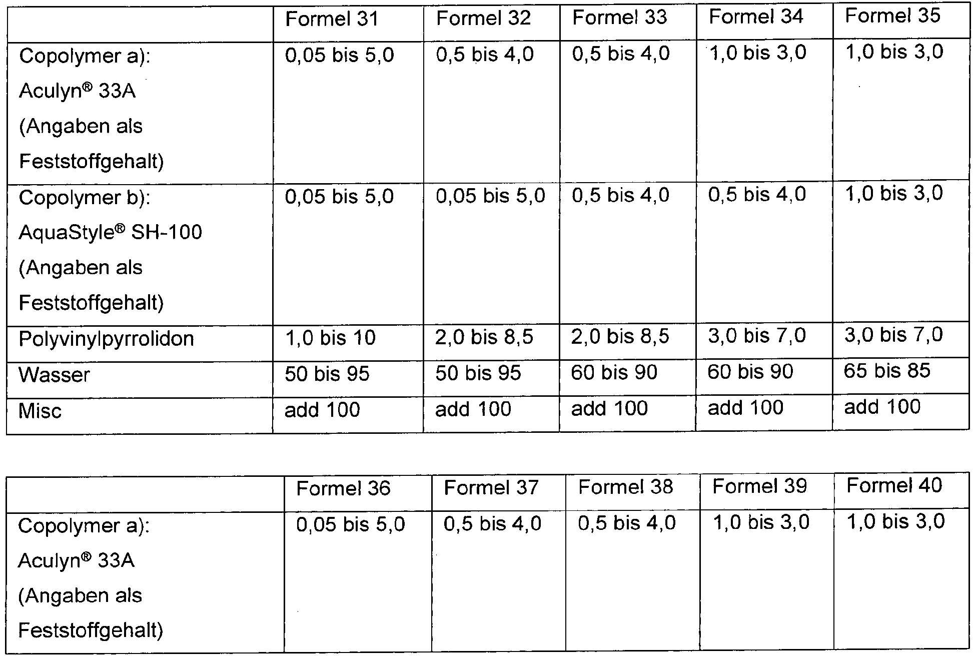

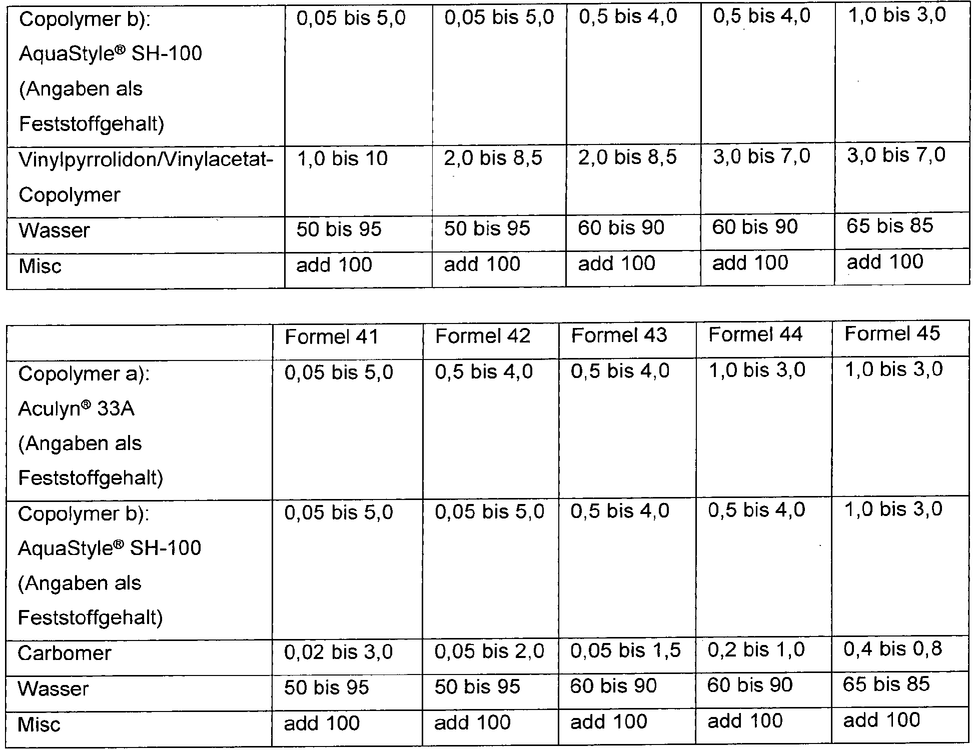

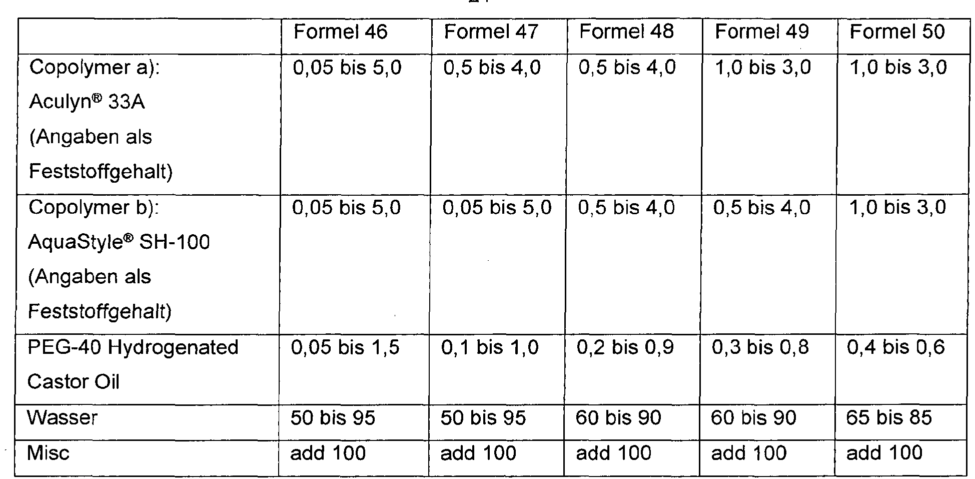

Unter "Misc" ist erfindungsgemäß ein kosmetischer Träger zu verstehen, insbesondere (sofern nicht separat angeführt) Wasser und ggf. weitere übliche Bestandteile von Stylingprodukten.

Die kosmetische Zusammensetzung der vorliegenden Erfindung kann in den für die temporäre Umformung von Haaren üblichen Formen konfektioniert sein, z. B. als Haargel, Haarspray Haarschaum oder Haarwachs. Bevorzugt ist die Konfektionierung als Haargel.

Sowohl Haarschäume als auch Haarsprays erfordern die Anwesenheit von Treibmitteln. Erfindungsgemäß sollten dafür jedoch bevorzugt keine oder nur geringe Mengen an Kohlenwasserstoffen eingesetzt werden. Propan, Propan/Butan-Gemische und Dimethylether sind erfindungsgemäß besonders geeignete Treibmittel.

Die vorliegende Erfindung betrifft auch die Verwendung von erfindungsgemäßen kosmetischen Zusammensetzungen zur temporären Umformung von keratinischen Fasern, insbesondere von menschlichen Haaren, sowie ein Verfahren zur temporären Verformung von keratinischen Fasern, insbesondere menschlichen Haaren, bei dem die erfindungsgemäße kosmetische Zusammensetzung auf keratinische Fasern appliziert wird.

Ein weiterer Gegenstand dieser Patentanmeldung ist die Verwendung einer erfindungsgemäßen kosmetischen Zusammensetzung zur Verbesserung der Feuchtebeständigkeit temporär verformter keratinischer Fasern.

### Beispiele

Es wurde folgende Haargele hergestellt:

| **Komponente/Rohstoff** | **INCI-Bezeichnung oder chemische Bezeichnung** | **V1** | **V2** | **E1** |
|---|---|---|---|---|
| Aculyn® A33 ¹ | Acrylates Copolymer | 3,58 | -- | 1,79 |
| AquaStyle SH-100² | Acrylates Copolymer (and) Water | -- | 3,3 | 1,65 |
| AMP-ULTRA PC 2000 | Aminomethyl Propanol | 0,24 | 0,3 | 0,27 |
| Wasser | | 96,18 | 96,4 | 96,29 |
| **Gesamt** | | 100 | 100 | 100 |

| | | | | |
|---|---|---|---|---|
| ¹ 28 Gew.-% Aktivsubstanz in Wasser ² 30 Gew.-% Aktivsubstanz in Wasser | | | | |

Die Mengenangaben in der Tabelle sind in Gew.-% des jeweiligen Rohstoffs, bezogen auf die gesamte Zusammensetzung, angegeben. Der Polymergehalt in jeder der Zusammensetzungen V1, V2 und E1 betrug 1,0 Gew.-%.

Für die erhaltenen Stylingmittel wurde mittels eines HHCR-Test (High Humidity Curl Retention-Test: 6h) an neuen Kerling-Haarsträhnen die Feuchtebeständigkeit bestimmt (Mittelwert bei Bestimmung an je 5 Haarsträhnen):

| | **V1** | **V2** | **E1** |
|---|---|---|---|
| HHCR | 63% | 51% | 71% |

Die erfindungsgemäße Polymerkombination E1 zeigte demnach einen deutlich überadditiven, synergistischen Effekt in Bezug auf die Feuchtebeständigkeit.

## Patentansprüche

1. Eine kosmetische Zusammensetzung zur temporären Umformung keratinischer Fasern, welche enthält:
(a) mindestens ein Acrylatcopolymer (a), welches zumindest aus folgenden Monomereinheiten aufgebaut ist:
(a1) mindestens eine (Meth)Acrylsäureeinheit
(a2) mindestens eine (Meth)Acrylsäureestereinheit, welche einen Acrylsäure-(C1-4)-alkylester aufweist,
und
(b) mindestens ein anionisches Acrylatcopolymer (b), welches nur aus den Einheiten (b1), (b2) und (b3) aufgebaut ist, wobei
(b1) mindestens eine (Meth)Acrylsäureeinheit, welche Methacrylsäure aufweist,
(b2) mindestens eine (Meth)Acrylsäureethylestereinheit, welche Ethylacrylat aufweist, und
(b3) mindestens eine (Meth)Acrylsäureestereinheit, die von der (Meth)Acrylsäureethylestereinheit (b2) verschieden und ein (Meth)Acrylsäurealkylester mit einer linearen oder verzweigten Alkylgruppe mit 2 bis 12 Kohlenstoffatomen ist,
aufweist.

2. Kosmetische Zusammensetzung nach Anspruch 1, wobei das mindestens eine Acrylatcopolymer (a) bezogen auf sein Gesamtgewicht zu mindestens 90 Gew.-%, vorzugsweise zu mindestens 95 Gew.-% und insbesondere zu mindestens 97 Gew.-% aus den Monomeren (a1) (Meth)Acrylsäureinheit und (a2) (Meth)Acrylsäureestereinheit besteht.

3. Kosmetische Zusammensetzung nach einem der vorhergehenden Ansprüche, wobei die Zusammensetzung das Copolymer (a) in einem Anteil von 0,05 bis 5,0 Gew.-%, bevorzugt 0,5 bis 4,0 Gew.-%, weiterhin bevorzugt 1,0 bis 3,0 Gew.-% enthält, bezogen auf das Gesamtgewicht der kosmetischen Zusammensetzung.

4. Kosmetische Zusammensetzung nach einem der vorhergehenden Ansprüche, wobei die Zusammensetzung das anionische Acrylatcopolymer (b) in einem Anteil von 0,05 bis 5,0 Gew.-%, bevorzugt 0,5 bis 4,0 Gew.-% und insbesondere von 1,0 bis 3,0 Gew.-% enthält, bezogen auf das Gesamtgewicht der kosmetischen Zusammensetzung.

5. Kosmetische Zusammensetzung einem der vorhergehenden Ansprüche, wobei die Zusammensetzung weiterhin mindestens ein von den Acrylatcopolymeren (a) und (b) verschiedenes Polymer (c), insbesondere ein anionisches oder nichtionisches Polymer (c), enthält.

6. Kosmetische Zusammensetzung nach einem der vorhergehenden Ansprüche, wobei die Zusammensetzung als Haargel, Haarspray, Haarschaum oder Haarwachs, insbesondere als Haargel, vorliegt.

7. Verwendung einer kosmetischen Zusammensetzung nach einem der Ansprüche 1 bis 6 zur temporären Umformung keratinischer Fasern.

8. Verfahren zur temporären Verformung von keratinischen Fasern, insbesondere menschlichen Haaren, bei dem die kosmetische Zusammensetzung nach einem der Ansprüche 1 bis 6 auf keratinische Fasern appliziert wird.

## Claims

1. A cosmetic composition for temporarily shaping keratin fibers, containing:
(a) at least one acrylate copolymer (a), which is made up of at least the following monomer units:
(a1) at least one (meth)acrylic acid unit,
(a2) at least one (meth)acrylic acid ester unit which comprises an acrylic acid (C1-4)-alkyl ester,
and
(b) at least one anionic acrylate copolymer (b), which is composed only of the units (b1), (b2) and (b3), wherein
(b1) comprises at least one (meth)acrylic acid unit which comprises methacrylic acid,
(b2) comprises at least one (meth)acrylic acid ethyl ester unit which comprises ethyl acrylate, and
(b3) comprises at least one (meth)acrylic acid ester unit which is different from the (meth)acrylic acid ethyl ester unit (b2) and is a (meth)acrylic acid alkyl ester which has a linear or branched alkyl group having 2 to 12 carbon atoms.

2. The cosmetic composition according to claim 1, wherein the at least one acrylate copolymer (a), based on the total weight thereof, consists of at least 90 wt.%, preferably at least 95 wt.% and in particular at least 97 wt.% of the monomers (a1) (meth)acrylic acid unit and (a2) (meth)acrylic acid ester unit.

3. The cosmetic composition according to one of the preceding claims, wherein the composition contains the copolymer (a) in a proportion of from 0.05 to 5.0 wt.%, preferably from 0.5 to 4.0 wt.%, particularly preferably from 1.0 to 3.0 wt.%, based on the total weight of the cosmetic composition.

4. The cosmetic composition according to one of the preceding claims, wherein the composition contains the anionic acrylate copolymer (b) in a proportion of from 0.05 to 5.0 wt.%, preferably from 0.5 to 4.0 wt.%, and in particular from 1.0 to 3.0 wt.%, based on the total weight of the cosmetic composition.

5. The cosmetic composition according to one of the preceding claims, wherein the composition further contains at least one polymer (c) which is different from acrylate copolymers (a) and (b), in particular an anionic or nonionic polymer (c).

6. The cosmetic composition according to one of the preceding claims, wherein the composition is a hair gel, hair spray, hair foam or hair wax, in particular a hair gel.

7. The use of a cosmetic composition according to one of claims 1 to 6 for temporarily shaping keratin fibers.

8. A method for temporarily shaping keratin fibers, in particular human hair, in which the cosmetic composition according to one of claims 1 to 6 is applied to keratin fibers.

## Revendications

1. Composition cosmétique pour la transformation temporaire des fibres kératiniques, comprenant :
(a) au moins un copolymère d'acrylate (a), qui est composé au moins des unités monomères suivantes :
(a1) au moins une unité d'acide (méth)acrylique,
(a2) au moins une unité d'ester d'acide (méth)acrylique comprenant un ester alkylique d'acide acrylique (C1-4)
et
(b) au moins un copolymère d'acrylate anionique (b) qui est constitué uniquement des motifs (b1), (b2) et (b3), où
(b1) au moins une unité d'acide (méth)acrylique comprenant de l'acide méthacrylique,
(b2) au moins une unité d'ester éthylique d'acide (méth)acrylique comprenant de l'acrylate d'éthyle, et
(b3) au moins une unité d'ester d'acide (méth)acrylique qui est différente de l'unité d'ester éthylique d'acide (méth)acrylique (b2) et qui est un ester alkylique d'acide (méth)acrylique ayant un groupe alkyle linéaire ou ramifié avec 2 à 12 atomes de carbone.

2. Composition cosmétique selon la revendication 1, dans laquelle l'au moins un copolymère d'acrylate (a) est composé à raison d'au moins 90 % en poids, de préférence d'au moins 95 % en poids et en particulier d'au moins 97 % en poids, par rapport à son poids total, des monomères (a1) motif acide (méth)acrylique et (a2) motif ester (méth)acrylique.

3. Composition cosmétique selon l'une quelconque des revendications précédentes, ladite composition contenant le copolymère (a) en une quantité de 0,05 à 5,0 % en poids, de préférence de 0,5 à 4,0 % en poids, de manière davantage préférée de 1,0 à 3,0 % en poids, par rapport au poids total de la composition cosmétique.

4. Composition cosmétique selon l'une quelconque des revendications précédentes, ladite composition contenant le copolymère anionique d'acrylate (b) dans une proportion de 0,05 à 5,0 % en poids, de préférence de 0,5 à 4,0 % en poids et en particulier de 1,0 à 3,0 % en poids, par rapport au poids total de la composition cosmétique.

5. Composition cosmétique selon l'une quelconque des revendications précédentes, ladite composition contenant en outre au moins un polymère (c) différent des copolymères d'acrylate (a) et (b), en particulier un polymère anionique ou non ionique (c).

6. Composition cosmétique selon l'une quelconque des revendications précédentes, ladite composition étant présente sous forme de gel capillaire, de laque, de mousse ou de cire pour cheveux, en particulier sous forme de gel capillaire.

7. Utilisation d'une composition cosmétique selon l'une des revendications 1 à 6 pour la mise en forme temporaire de fibres kératiniques.

8. Procédé de mise en forme temporaire de fibres kératiniques, en particulier de cheveux humains, dans lequel la composition cosmétique selon l'une des revendications 1 à 6 est appliquée sur des fibres kératiniques.
